# EUROPEAN PATENT SPECIFICATION

(11) **EP 3 130 359 B1**
(45) Date of publication and mention of the grant of the patent: **01.08.2018**
(21) Application number: 16173826.5
(22) Date of filing: 09.06.2016
(51) Int. Cl.: A61L 2/20, A61L 2/26, B65B 55/10, B67C 7/00

(54) **STERILISATION APPARATUS FOR STERILISING CONTAINERS**
STERILISATIONSVORRICHTUNG ZUR STERILISIERUNG VON BEHÄLTERN
APPAREIL DE STÉRILISATION POUR STÉRILISER DES CONTENEURS

(30) Priority: 31.07.2015 IT UB20152718
(43) Date of publication of application: 15.02.2017
(73) Proprietor: Gea Procomac S.p.A., 43038 Sala Baganza (PR) (IT)
(72) Inventor: ABELLI, Paolo, 43038 SALA BAGANZA (PARMA) (IT); COMANI, Andrea, 43038 SALA BAGANZA (PARMA) (IT)
(74) Representative: Dondi, Silvia

(56) References cited:
- EP-A1- 2 455 106
- EP-A1- 2 604 295
- EP-A1- 2 803 628
- JP-A- 2005 065 882

## Description

The present invention relates to an apparatus for sterilising containers.

The reference sector is the bottling of so-called "sensitive" food products, i.e. products that are particularly sensitive to bacteriological contamination and oxidation, such as, for example, isotonic drinks, juices, nectars, soft drinks, teas, milk-based drinks, coffee-based drinks, etc., for which it is fundamental to prevent any microbiological contamination throughout the packaging stages.

In this context, the focus is on the decontamination of the primary packaging with which the product comes into contact, whether it be a bottle (or a preform), a pouch or flexible container or a cardboard container (brick).

In particular, the present invention relates to aseptic packaging of beverages in bottles made of thermoplastic material (preferably PET), the preform of which is sterilised. The sterile preform is subsequently blown under sterile conditions, in such a way as to obtain a sterile bottle ready to be filled directly, without any intermediate sterilisation or rinsing.

The decontamination techniques known to date provide for the use of chemical agents (e.g. aqueous solutions containing peracetic acid or hydrogen peroxide), the delivery of radiation or the use of heat.

The chemical agents commonly used are peracetic acid and hydrogen peroxide. In the present description and in the appended claims, the term "hydrogen peroxide" relates to an aqueous solution of hydrogen peroxide, preferably at 35%.

The application of hydrogen peroxide generally takes place according to two methods.

A first method for chemically sterilising the walls of a container comprises the following steps:
- vaporising an aqueous solution containing hydrogen peroxide inside the container;
- causing the hydrogen peroxide vapours to condense on the inner walls of the container (by bringing the walls themselves to a temperature equal to or lower than the temperature of condensation of the peroxide vapours);

- causing the condensate to evaporate, for example with hot air, so as to "activate" the previously condensed hydrogen peroxide;
- drying the inner walls of the container.

A second chemical sterilisation method envisages keeping the hydrogen peroxide constantly in the vapour state. The hydrogen peroxide is vaporised and the vapours are delivered onto the surface to be sterilised. As this surface has a temperature that is higher than the temperature of condensation of the peroxide vapours, no condensation takes place at any time during the sterilisation period. In this case, given the temperature of the vapours, it is not necessary to activate the hydrogen peroxide.

Both methods comprise a step in which an aqueous solution of hydrogen peroxide is brought from the liquid phase to the gaseous phase, called VHP (Vapour Hydrogen Peroxide).

A known way to generate VHP is to drip liquid hydrogen peroxide onto a hot plate (for example, heated with electrical heating elements) having a temperature that is sufficient to cause the hydrogen peroxide to evaporate. It is envisaged that another fluid will be fed into the plate vaporiser, typically air, which acts as a medium, i.e. it has the task of delivering the hydrogen peroxide vapours generated towards the surface to be sterilised. An example of this type of vaporiser is disclosed in patent EP2455106. Ideally, the vaporiser apparatus must be positioned as close as possible to the area in which the decontamination takes place. In fact, it is fundamental that the hydrogen peroxide evaporates completely and remains in a gaseous phase until the time of sterilisation. The complete evaporation of the hydrogen peroxide makes it possible to maintain control of the process over time in such a way as to control the concentration of the sterilising agent on the surface to be treated.

In the process it is in fact necessary to provide for the correct hourly amount of liquid hydrogen peroxide to be vaporised, which depends both on the desired concentration of the gaseous phase inside the fluid medium (tied to the required sterilisation performance and dependent on the type of product filled) and on the flow rate of the fluid medium (tied to how many containers need to be sterilised per hour, thus on the size of the sterilizing machine).

If, in some point of the system, between vaporisation and sterilisation, the hydrogen peroxide vapours were to condense, even in small amounts, the concentration of the sterilising agent would decrease and there would be a loss of control over the process, resulting in conditions in which the decontamination might not be effective.

In controlling the process, it is also necessary to maintain the temperature of the hydrogen peroxide vapours above a minimum value in order to ensure the effectiveness of sterilisation. This aspect is of particular importance in the production of low-acid beverages, which require a superior performance in terms of packaging sterilisation compared to other beverages.

A fundamental requirement in the sterilisation of packaging intended to contain beverages is that the sterilising effect must be greater than/equal to the established value and maintained constant over time.

In order to obtain this condition it is important, as noted above, that no condensate is formed and that the temperature of the vapours is above the threshold value. The two phenomena are correlated: if the system can be successfully maintained above the dew point temperature, the formation of "cold" zones where the vapours can condense will be less likely.

Document JP 2005065882 discloses the provision of a heater with inclined surface to minimise surface temperature changes and thus provide stable vapor concentration.

EP 2604295 and EP 2803628 discloses suitable transport mechanism of a gaseous sterilant to containers to be sterilised by means of rotary vapor distributors comprising a fixed and a rotating part.

In this context, the technical task at the basis of the present invention is to propose an apparatus for sterilising containers which overcomes the aforementioned drawbacks of the prior art.

In particular, the object of the present invention is to propose an apparatus for sterilising containers which assures that the desired degree of sterility is obtained and maintained over time, without having to increase the structural complexity and/or overall dimensions.

The stated technical task and specified objects are substantially achieved by an apparatus for sterilising containers comprising:
- a vaporisation chamber having a first inlet for a gaseous fluid and at least a second inlet for an aqueous composition containing a sterilising agent, the vaporisation chamber being configured to generate a mixture formed by the gaseous fluid, water vapour and evaporated sterilising agent;
- a rotary fluid dispenser having a fixed portion and a rotating portion rotating about a predefined axis of rotation, the rotating portion bearing a plurality of dispensing nozzles, wherein the vaporisation chamber is obtained in the fixed portion of the rotary dispenser and is in fluid communication with the dispensing nozzles so as to feed them with the mixture.

In particular, the vaporisation chamber is bounded in the upper part and laterally by walls of the fixed portion.

In a preferred embodiment, the vaporisation chamber is bounded in the lower part by a lower wall of the fixed portion. The lower wall is made at least partially of metal.

There are also provided electrical heating elements operatively active on the lower wall in such a way as to heat it.

Preferably, the sterilisation apparatus comprises a doser associated with the second inlet and configured to drip the aqueous composition on the lower wall.

Preferably, the lower wall has conical shape converging in a point located on the predefined axis of rotation.

The sterilisation apparatus further comprises a level sensor located in the vaporisation chamber in proximity to the point of convergence of the lower wall 5a. The level sensor is configured to detect the level of non-evaporated aqueous composition which is present in the vaporisation chamber.

Preferably, the first inlet of the vaporisation chamber consists in an opening or hole fashioned in the centre of an upper wall of the fixed portion.

In a preferred embodiment, the fixed portion comprises a first disc, on top of which there is a hollow main body that contains the vaporisation chamber and is coaxial with the first disc. The rotating portion in turn comprises a second disc located below the first disc and coaxial with the latter and with the main hollow body.

Preferably, the first disc is provided with an arched window whose angular position relative to the predefined axis of rotation can be adjusted by varying the initial relative position between the first disc and the second disc.

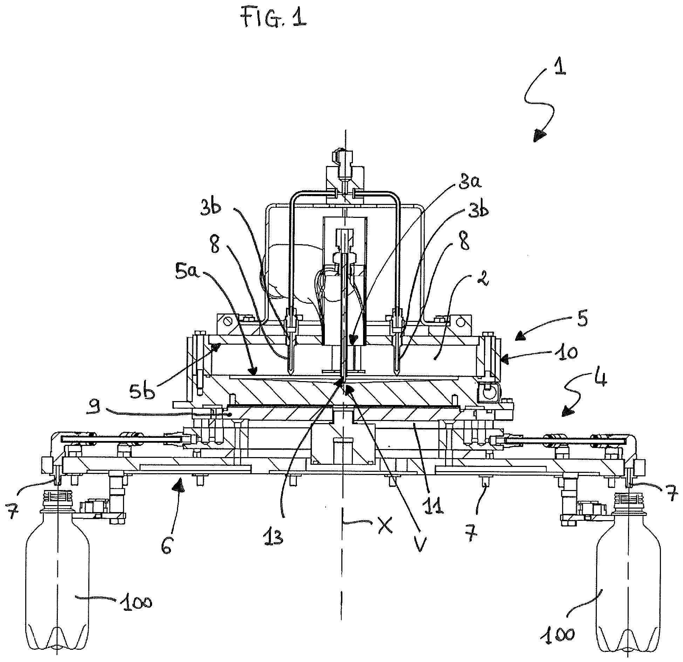

Further features and advantages of the present invention will be more apparent from the approximate, and hence non-limiting description of a preferred, but not exclusive, embodiment of an apparatus for sterilising containers, as illustrated in figure 1, which represents a sectional view of the apparatus for sterilising containers according to the present invention. With reference to figure 1, the number 1 indicates an apparatus for sterilising containers 100.

In particular, the containers 100 are preforms or bottles made of polyethylene (known by the acronym PET, which stands for polyethylene terephthalate) or high-density polyethylene (known by the acronym HDPE).

Alternatively, the containers 100 are pouches or flexible containers, or cardboard containers.

The sterilisation apparatus 1 comprises a vaporisation chamber 2 having a first inlet 3a for a gaseous fluid (preferably air or water vapour) and at least a second inlet 3b for an aqueous composition containing a sterilising agent.

For example, the sterilising agent is liquid hydrogen peroxide. Preferably, the concentration of hydrogen peroxide in the aqueous composition is about 35%.

The vaporisation chamber 2 is configured to generate a mixture formed by the gaseous fluid (for example air or water vapour), water vapour and the evaporated sterilising agent.

The sterilisation apparatus 1 comprises a rotary fluid dispenser 4 having a fixed portion 5 and a rotating portion 6 rotating about a predefined axis of rotation X. The rotating portion 6 bears a plurality of dispensing nozzles 7. Advantageously, the vaporisation chamber 2 is obtained in the fixed portion 5 of the rotary dispenser 4 and is in fluid communication with the dispensing nozzles 7 of the rotating portion 6 so as to feed them with the mixture.

In other words, the vaporisation chamber 2 is incorporated into the structure of the rotary fluid dispenser 4.

Preferably, the vaporisation chamber 2 is bounded in the upper part and laterally by walls of the fixed portion 5 of the rotary dispenser 4. Preferably, the first inlet 3a of the vaporisation chamber 2 consists in an opening or hole fashioned in the centre of an upper wall 5b of the fixed portion 5.

Preferably, the vaporisation chamber 2 is bounded in the lower part by a lower wall 5a made at least partially of metal. There are provided electrical heating elements (not illustrated) operatively active on the lower wall 5a of the fixed portion 5 so as to heat it.

Preferably, associated with the inlet 3b of the vaporisation chamber 2 there is a doser 8 configured to drip the aqueous composition on the lower wall 5a.

In other words, the lower wall 5a acts as a plate that can be heated until reaching a temperature that is sufficient to cause the sterilising agent present in the aqueous composition to evaporate.

In an unillustrated alternative, instead of having heating elements, the lower wall 5a is shaped in such a way as to circulate vapour, thus veritably creating a vapour heat exchanger.

Advantageously, the lower wall 5a of the vaporisation chamber 2 has a conical shape converging in a point or vertex V located on the predefined axis of rotation X.

In other words, the lower wall 5a delimits a funnel-shaped conical volume. Preferably, the fixed portion 5 of the rotary dispenser 4 comprises a first disc 9, on top of which there is a hollow main body 10 containing the vaporisation chamber 2. The first disc 9 and the hollow main body 10 are coaxial.

For example, the hollow main body 10 is substantially cylindrical and the lower base thereof represents the lower wall 5a of the vaporisation chamber 2. The internal lateral surface of the hollow main body 10 laterally bounds the vaporisation chamber 2. The upper internal base of the hollow main body 10 bounds the upper part of the vaporisation chamber 2.

Given that in the embodiment described and illustrated here the lower wall 5a has a conical shape, it follows that the hollow main body 10 is funnel shaped in the lower part.

As can be seen in figure 1, the hollow main body 10 and the first disc 9 are coaxial; their axis is represented by the predefined axis of rotation X.

The rotating portion 6 comprises at least a second disc 11 coaxial with the first disc 9 and located below and in contact with the latter.

Preferably, the first disc 9 is provided with an arched window (or slot), i.e. an opening passing through one base to the other of the first disc 9. In particular, the arched window extends along an arc subtending an angle of less than 340°.

Preferably, the initial relative position between the first disc 9 and the second disc 11 can be adjusted to vary the initial angular position of the arched window relative to the predefined axis of rotation X.

Initial angular position means the position taken on by the arched window relative to the predefined axis of rotation X before the rotary fluid dispenser 4 starts operating.

An example of a rotary dispenser with adjustment of the initial angular position of the arched window is described in patent EP2614031 in the name of the Applicant. Preferably, the sterilisation apparatus 1 comprises a level sensor 13 operatively active on the vaporisation chamber 2 in order to detect the level of non-evaporated aqueous composition. Advantageously, the level sensor 13 is located in the vaporisation chamber 2 at the point of convergence V of the lower wall 5a.

The level sensor 13 is of a known type and will thus not be further described.

The operation of the sterilisation apparatus for sterilising containers, according to the present invention, is described below.

Air (or another gaseous fluid) is centrally introduced into the vaporisation chamber 2 through the first inlet 3a. The air acts as a "carrier", i.e. a medium for delivering the gaseous compound that is generated by the vaporisation of the injected aqueous composition and dripped into the vaporisation chamber 2 by means of the doser 8. In the embodiment illustrated in figure 1, two dosers 8 can be seen (there are four in all) which inject microdrops of aqueous composition containing the sterilising agent. As the lower wall 5a, which acts as a plate, is heated, the aqueous composition evaporates and the mixture of air, water vapour and evaporated sterilising agent is thus obtained.

This mixture is fed to the nozzles 7, which dispense it towards the containers 100 to be sterilised.

The concentration of sterilising agent in the gaseous mixture is maintained within a desired interval via a feedback control (not illustrated).

From the description given, the features of the apparatus for sterilising containers according to the present invention appear clear, as do the advantages thereof.

In particular, as the vaporisation chamber is obtained inside the fixed portion of the rotary fluid dispenser, the rotary dispenser produced also acts as a vaporiser, resulting, therefore, in a compact sterilisation apparatus which produces and dispenses the vapours of the sterilising agent. This compactness has an impact not only on the overall dimensions, but also on the control of the process, since the evaporated sterilising agent is immediately fed to the dispensing nozzles without passing through "dead" sections in which it would risk condensing partially. Moreover, the conical shape of the heating plate causes the liquid residues of the sterilising agent to drain toward the point of convergence, where they collect and where the level sensor that contributes to controlling the efficiency of the vaporisation process is located.

The introduction of the gaseous fluid (air) centrally to the vaporisation chamber also contributes to efficiency.

## Claims

1. Sterilisation apparatus (1) for sterilising containers (100), comprising:
a vaporisation chamber (2) having a first inlet (3a) for a gaseous fluid and at least one second inlet (3b) for a watery composition containing a sterilising agent, said vaporisation chamber (2) begin configured to generate a mixture formed by the gaseous fluid, water vapour and evaporated sterilising agent;
a rotary fluid dispenser (4) having a fixed portion (5) and a rotating portion (6) rotating about a predefined axis of rotation (X), said rotating portion (6) bearing a plurality of dispensing nozzles (7), said vaporisation chamber (2) being in fluid communication with said dispensing nozzles (7) so as to feed them with said mixture,
**characterised in that** said vaporisation chamber (2) is obtained within the fixed portion (5) of said rotary dispenser (4).

2. Sterilisation apparatus (1) according to claim 1, wherein said vaporisation chamber (2) is bounded in the upper part and laterally by walls of said fixed portion (5).

3. Sterilisation apparatus (1) according to claim 2, wherein said vaporisation chamber (2) is bounded in the lower part by a lower wall (5a) of said fixed portion (5), said lower wall (5a) being made at least partially by metal.

4. Sterilisation apparatus (1) according to claim 3, further comprising electrical resistances that are operatively active on said lower wall (5a) in such a way as to heat it.

5. Sterilisation apparatus (1) according to claim 4, comprising a doser (8) associated to said at least one second inlet (3b) and configured to drip the watery composition on said lower wall (5a).

6. Sterilisation apparatus (1) according to claims 3 to 5, wherein said lower wall (5a) has a conical development that converges into a point (V) located on said predefined axis of rotation (X).

7. Sterilisation apparatus (1) according to claim 6, further comprising a level sensor (13) arranged in said vaporisation chamber (2) at the point (V) of convergence of said lower wall (5a), said level sensor (13) being configured to detect the level of non-evaporated watery composition which is present said vaporisation chamber (2).

8. Sterilisation apparatus (1) according to any of the previous claims, wherein said first inlet (3a) consists in an opening or hole obtained in the center of an upper wall (5b) of said fixed portion (5).

9. Sterilisation apparatus (1) according to any of the preceding claims, wherein said fixed portion (5) comprises a first disc (9) upon which is arranged a hollow main body (10) that contains said vaporisation chamber (2) and is coaxial to said first disc (9), said rotating portion (6) comprising a second disc (11) located below said first disc (9) and coaxial to the latter and to said hollow main body (10).

## Patentansprüche

1. Sterilisationsvorrichtung (1) zur Sterilisierung von Behältern (100), umfassend:
eine Verdampfungskammer (2) mit einem ersten Einlass (3a) für ein gasförmiges Fluid und mindestens einem zweiten Einlass (3b) für eine wässrige Zusammensetzung, die ein Sterilisierungsmittel enthält, wobei die Verdampfungskammer (2) so konfiguriert ist, dass sie ein Gemisch aus dem gasförmigen Fluid, Wasserdampf und verdampftem Sterilisierungsmittel erzeugt;
einen rotierenden Fluidspender (4) mit einem festen Abschnitt (5) und einem rotierenden Abschnitt (6), der sich um eine vorbestimmte Drehachse (X) dreht, wobei der rotierende Abschnitt (6) eine Vielzahl von Spenderdüsen (7) trägt, wobei die Verdampfungskammer (2) in Fluidverbindung mit den Spenderdüsen (7) steht, um sie mit dem Gemisch zu versorgen,
**dadurch gekennzeichnet, dass** die Verdampfungskammer (2) innerhalb des festen Abschnitts (5) des Drehspenders (4) erhalten wird.

2. Sterilisationsvorrichtung (1) nach Anspruch 1, wobei die Verdampfungskammer (2) im oberen Teil und seitlich durch Wände des festen Abschnitts (5) begrenzt wird.

3. Sterilisationsvorrichtung (1) nach Anspruch 2, wobei die Verdampfungskammer (2) im unteren Teil durch eine untere Wand (5a) des festen Abschnitts (5) begrenzt wird, wobei die untere Wand (5a) mindestens teilweise
aus Metall ausgebildet ist.

4. Sterilisationsvorrichtung (1) nach Anspruch 3, umfassend ferner elektrische Widerstände, die an der unteren Wand (5a) derart wirksam sind, dass sie diese erwärmen.

5. Sterilisationsvorrichtung (1) nach Anspruch 4, umfassend einen Dosierer (8), der mindestens dem einen zweiten Einlass (3b) zugeordnet ist und so konfiguriert ist, dass er die wässrige Zusammensetzung auf die untere Wand (5a) tropft.

6. Sterilisationsvorrichtung (1) nach den Ansprüchen 3 bis 5, wobei die untere Wand (5a) eine konische Entwicklung aufweist, die in einen Punkt (V) konvergiert, der sich auf der vorbestimmten Drehachse (X) befindet.

7. Sterilisationsvorrichtung (1) nach Anspruch 6, die ferner einen Niveausensor (13) umfasst, der in der Verdampfungskammer (2) an dem Punkt (V) der Konvergenz der unteren Wand (5a) angeordnet ist, wobei der Niveausensor (13) so konfiguriert ist, dass er das Niveau der nicht verdampften wässrigen Zusammensetzung erfasst, die in der Verdampfungskammer (2) vorhanden ist.

8. Sterilisationsvorrichtung (1) nach einem der vorhergehenden Ansprüche, wobei der erste Einlass (3a) aus einer Öffnung oder einem Loch besteht, die in der Mitte einer oberen Wand (5b) des festen Abschnitts (5) erhalten wird.

9. Sterilisationsvorrichtung (1) nach einem der vorhergehenden Ansprüche, wobei der feste Abschnitt (5) eine erste Scheibe (9) umfasst, auf der ein hohler Hauptkörper (10) angeordnet ist, der die Verdampfungskammer (2) enthält und koaxial zu der ersten Scheibe (9) ist, wobei der rotierende Abschnitt (6) eine zweite Scheibe (11) umfasst, die sich unterhalb der ersten Scheibe (9) und koaxial zu dieser und dem hohlen Hauptkörper (10) befindet.

## Revendications

1. Appareil de stérilisation (1) pour stériliser des conteneurs (100), comprenant :
une chambre de vaporisation (2) avec une première entrée (3a) pour un fluide gazeux et au moins une seconde entrée (3b) pour une composition aqueuse contenant un agent de stérilisation, ladite chambre de vaporisation (2) étant configurée pour générer un mélange composé de fluide gazeux, de vapeur d'eau et d'agent de stérilisation évaporé ;
un distributeur rotatif (4) de fluide avec une partie fixe (5) et une partie rotative (6) pivotant autour d'un axe de rotation prédéfini (X), ladite partie rotative (6) supportant une pluralité de buses de distribution (7), ladite chambre de vaporisation (2) étant en communication fluidique avec lesdites buses de distribution (7) de sorte à les alimenter avec ledit mélange,
**caractérisé en ce que** ladite chambre de vaporisation (2) est réalisée à l'intérieur de la partie fixe (5) dudit distributeur rotatif (4).

2. Appareil de stérilisation (1) selon la revendication 1, dans lequel ladite chambre de vaporisation (2) est délimitée dans la partie supérieure et latéralement par les parois de ladite partie fixe (5).

3. Appareil de stérilisation (1) selon la revendication 2, dans lequel ladite chambre de vaporisation (2) est délimitée dans la partie inférieure par une paroi inférieure (5a) de ladite partie fixe (5), ladite paroi inférieure (5a) étant faite au moins partiellement
en métal.

4. Appareil de stérilisation (1) selon la revendication 3, comprenant également des résistances électriques qui sont opérationnellement actives sur ladite paroi inférieure (5a) pour la chauffer.

5. Appareil de stérilisation (1) selon la revendication 4, comprenant un doseur (8) associé à ladite au moins une seconde entrée (3b) et configuré pour laisser tomber goutte à goutte la composition aqueuse sur ladite paroi inférieure (5a).

6. Appareil de stérilisation (1) selon les revendications 3 à 5, dans lequel ladite paroi inférieure (5a) présente un développement conique qui converge en un point (V) situé sur ledit axe de rotation prédéfini (X).

7. Appareil de stérilisation (1) selon la revendication 6, comprenant également un capteur de niveau (13) placé dans ladite chambre de vaporisation (2) au point (V) de convergence de ladite paroi inférieure (5a), ledit capteur de niveau (13) étant configuré pour détecter le niveau de composition aqueuse non évaporée dans ladite chambre de vaporisation (2).

8. Appareil de stérilisation (1) selon l'une quelconque des revendications précédentes, dans lequel ladite première entrée (3a) consiste en une ouverture ou un trou se trouvant dans le centre d'une paroi supérieure (5b) de ladite partie fixe (5).

9. Appareil de stérilisation (1) selon l'une quelconque des revendications précédentes, dans lequel ladite partie fixe (5) comprend un premier disque (9) surmonté d'un corps principal (10) creux contenant ladite chambre de vaporisation (2) et coaxial audit premier disque (9), ladite partie rotative (6) comprenant un second disque (11) situé en dessous dudit premier disque (9) et coaxial à ce dernier et audit corps principal (10) creux.
